# EUROPÄISCHE PATENTSCHRIFT

(11) **EP 0 511 269 B1**
(45) Veröffentlichungstag und Bekanntmachung des Hinweises auf die Patenterteilung: **19.04.1995**
(21) Anmeldenummer: 91902686.4
(22) Anmeldetag: 07.01.1991
(51) Int. Cl.: B08B 3/08, C11D 1/40, C11D 3/30, C10M 133/04

(54) **LEERGUTÜBERDUSCHUNG**
OVERSPRAYING OF EMPTIES
NETTOYAGE D'EMBALLAGES VIDES

(30) Priorität: 16.01.1990 DE 4000982
(43) Veröffentlichungstag der Anmeldung: 04.11.1992
(73) Patentinhaber: Henkel Kommanditgesellschaft auf Aktien, 40191 Düsseldorf (DE)
(72) Erfinder: FALTER, Wolfgang, B-4731 Lichtenbusch (BE); SCHMITZ, Karl-Heinz, W-4006 Erkrath (DE)
(86) Internationale Anmeldenummer: EP9100017
(87) Internationale Veröffentlichungsnummer: WO9110514

(56) Entgegenhaltungen:
- WO-A-90/10053
- DE-A- 3 631 953
- DE-A- 3 905 548
- US-A- 4 683 008

## Beschreibung

Die vorliegende Erfindung betrifft ein Verfahren zur Desinfektion und Reinigung in der Lebensmittel- und Getränkeindustrie unter Verwendung von an sich bekannten Gleitmitteln auf Basis von sekundären und/oder tertiären Aminen und/oder Salzen derartiger Amine und gegebenenfalls üblichen Verdünnungsmitteln oder Hilfs- beziehungsweise Zusatzstoffen zur Überduschung von Leergut.

Leergut im Sinne der vorliegenden Erfindung sind vorzugsweise Getränkeflaschen, Glas- und Kunststoffflaschen, Dosen, Gläser, Fässer und Getränkecontainer (KEG).

Die Hygieneketten in der Ernährungsindustrie werden durch verlängerte Distributions- und Verteilwege immer länger. Daher muß der Hygiene bei der Verarbeitung und Verpackung von Lebensmitteln immer mehr Bedeutung zugemessen werden. Besonders problematisch sind Mehrwegsysteme, wie sie z.B. in der Getränkeindustrie schon lange weltweit praktiziert werden, da von dem zurückgenommenen Leergut eine große Infektionsgefahr ausgeht. Durch die Übertragung von Mikroorganismen aus der Luft des Abfüll- und Verpackungsraums auf das gereinigte Leergut und auf den Maschinenpark, insbesondere die Abfüllmaschine selbst, kann das abzufüllende Nahrungsmittel infiziert werden.

Desinfektionsmaßnahmen an der Abfüllmaschine selbst sind während des Betriebs problematisch, da das Desinfektionsmittel die Originalität des Nahrungsmittels beeinträchtigen kann. Es müssen daher Vorbeugungsmaßnahmen getroffen werden, um möglichst wenig Keimmaterial von außen in den Abfüllprozeß zu schleppen. Sinnvoll ist daher eine Reduktion des Keimgehaltes schon an dem Ort, wo das infizierte Leergut in den Abfüllraum geführt wird.

Zu diesem Zwecke sind Leergutüberdüsungssysteme zur Reduktion des Luftkeimgehaltes bekannt. Es wird ein Wasser-Desinfektionsmittelgemisch auf das in die Abfüllhalle einkommende Leergut gesprüht. Die Zeit, die das Desinfektionsmittel besitzt, bis es in der zentralen Reinigungsmaschine wieder abgeduscht wird, ist ausreichend, um eine deutliche Keimreduktion zu erzielen. Die anhaftenden Desinfektionsmittelreste werden dann durch die Flaschenwäsche wieder vollständig entfernt. Technisch vermeidbare Desinfektionsmittelrückstände sind somit ausgeschlossen.

Obwohl diese Idee sicher eine hygienische Teilmaßnahme darstellt, hat dieses Konzept seit seiner Einführung keine große Akzeptanz gefunden. Prinzipielle Nachteile des Standes der Technik zur Leergutüberduschung sind:
- sehr hoher apparativer Aufwand;
- Verschlechterung der Arbeitssicherheit durch zusätzliches Desinfektionsmittel direkt am Arbeitsplatz (Arbeitssicherheit - handling von Kanistern, Verwechslungsgefahr, MAK-Werte);
- Verschlechterung des Prozeßablaufs durch Reaktion des Desinfektionsmittels mit Inhaltsstoffen der Additive in der Reinigungsmaschine;
- Verschlechterung der Gesamtökologie durch zusätzliche Abwasserbelastung durch mittel bis stark wassergefährdende Desinfektionsmittel;
- im Verhältnis zum Aufwand geringer Effekt.

Aufgabe des erfindungsgemäßen Verfahrens ist es, diese Nachteile des derzeitigen Standes der Technik mit den positiven Hygiene-Aspekten der Leergutüberduschung zu verknüpfen.

In der US-A-4 683 008 wird eine Formulierung zur Reinigung harter Oberflächen beschrieben, das insbesondere im Sprühverfahren Verwendung finden kann. Diese alkalischen Reinigerformulierungen enthalten alkoxylierte nichtionische Tenside, anionische Tenside, organische und/oder anorganische Buildersalze sowie spezielle wasserlösliche Aminoverbindungen. Bei diesen Aminoverbindungen handelt es sich um Alkanolamide höherer Alkansäuren und/oder um Aminoxide. Diese Aminoverbindungen dienen hierbei dazu, die Reinigungskraft derartiger Mittel zu verstärken sowie ein längeres Anhaften dieser Mittel insbesondere an senkrechten Oberflächen zu bewirken.

Die vorliegende Erfindung betrifft demgegenüber ein Verfahren zur Desinfektion in der Lebensmittel- und Getränkeindustrie unter Verwendung von Aminen, dadurch gekennzeichnet, daß man ein an sich bekanntes Gleitmittel auf Basis von sekundären und/oder tertiären Aminen und/oder Salzen derartiger Amine und gegebenenfalls üblichen Verdünnungsmitteln oder Hilfs- bzw. Zusatzstoffen zur Überduschung von Leergut verwendet, welches
(a) mindestens ein sekundäres Amin der allgemeinen Formeln (Ia) oder (Ib)

   R-NH-R¹ (Ia)

   R-N⁺H₂-R¹ X⁻ (Ib)

   und/oder
(b) mindestens ein sekundäres Diamin der allgemeinen Formeln (IIa), (IIb) oder (IIc)

   R-NH-(CH₂)₃NH₂ (IIa)

   R-NH-(CH₂)₃N⁺H₃ X⁻ (IIb)

   R-N⁺H₂-(CH₂)₃-N⁺H₃ 2X⁻ (IIc)

   und/oder
(c) mindestens ein tertiäres Amin der allgemeinen Formeln (IIIa) oder (IIIb)

   R-NR³R⁴ (IIIa)

   R-N⁺HR³R⁴ X⁻ (IIIb)

enthält, wobei die Reste R und R¹ jeweils unabhängig voneinander bedeuten:
- einen substituierten oder unsubstituierten, linearen oder verzweigten, gesättigten oder einfach oder mehrfach ungesättigten Alkylrest mit 6 bis 22 C-Atomen, der als Substituenten mindestens einen Amin-, Imin-, Hydroxy-, Halogen- und/oder Carboxyrest aufweisen kann,
- einen substituierten oder unsubstituierten Phenylrest, der als Substituenten mindestens einen Amin-, Imin-, Hydroxy-, Halogen-, Carboxy- und/oder einen linearen oder verzweigten, gesättigten oder einfach oder mehrfach ungesättigten Alkylrest mit 6 bis 22 C-Atomen aufweisen kann,
   und X⁻ ein Anion aus der Gruppe Amidosulfonat, Nitrat, Halogenid, Sulfat, Hydrogencarbonat, Carbonat, Phosphat oder

   R²-COO⁻

bedeutet, wobei der Rest R² für
- Wasserstoff,
- einen substituierten oder unsubstituierten, linearen oder verzweigten Alkylrest mit 1 bis 20 C-Atomen oder Alkenylrest mit 2 bis 20 C-Atomen, die als Substituenten mindestens einen Hydroxy-, Amin- oder Iminrest aufweisen können, oder
- einen substituierten oder unsubstituierten Phenylrest, der als Substituenten einen Alkylrest mit 1 bis 20 C-Atomen aufweisen kann,
steht, und wobei die Reste R³ und R⁴ jeweils unabhängig voneinander bedeuten:
- einen substituierten oder unsubstituierten, linearen oder verzweigten Alkylrest mit 1 bis 20 C-Atomen oder Alkenylrest mit 2 bis 20 C-Atomen, die als Substituenten mindestens einen Hydroxy-, Amin- oder Iminrest aufweisen können, oder
- einen substituierten oder unsubstituierten Phenylrest, der als Substituenten einen Alkylrest mit 1 bis 20 C-Atomen aufweisen kann,
und wobei der Anteil der Amine an der Gesamtformulierung 1 bis 100 Gew.-% beträgt.

Zu diesem Zweck kann dann das normale Sprüh- und Verteilsystem für Kettengleitmittel erweitert werden. Derartige Kettengleitmittel sind in der älteren deutschen Patentanmeldung DE-A-39 05 548 beschrieben. Diese eignen sich in der für die Transportbandschmierung erforderlichen Konzentration auch für die Leergutüberduschung. Die Wirksamkeit dieser Mittel ist überraschenderweise nicht nur auf die Applikation in zentralen Transportbandsystemen beschränkt. Bei Anwendung zur Leergutüberduschung zeigten sich gegenüber den herkömmlichen Alternativen große Verfahrensvorteile.

Zur Anwendung der Kettengleitmittellösung zur Leergutüberduschung sei die notwendige Ausführung beispielhaft an einem Flaschenkeller der Getränkeindustrie erläutert. Das in den Flaschenkeller auf Paletten hereinkommende Leergut wird depalettiert. Die so vereinzelten Getränkekästen mit schmutzigem Leergut können über separate Düsenstöcke, die an das vorhandene zentrale Schmiermittelsystem angeflanscht sind, beaufschlagt werden. Vorteilhaft für Getränkekästen hat sich eine versetzte Anordnung von zwei Düsenstöcken bewährt. Diese sind mit jeweils drei Düsen zur Überduschung und einem Durchsatz von ca. 20 l/h zur Überschwallung der Kästen und des Mündungsbereichs der Flaschen und mit je zwei Düsen zur seitlichen Besprühung der Kästen ausgestattet. Die seitlichen Düsen benötigen eine geringere Kapazität von etwa 8 bis 10 l/h. Somit ergibt sich ein Gesamtverbrauch von etwa 160 l/h je Kastenlinie. Bei einer Konzentration von etwa 0,2 bis 0,4 %, bezogen auf übliche Schmiermittel- bzw. Desinfektionsmittelkonzentrate, die beispielsweise 15 Gew.-% Wirksubstanz enthalten, ergibt sich insgesamt ein sehr geringer Chemikalienbedarf von nur 0,32 bis 0,64 l/h.

Zu beachten ist gegebenenfalls die Dosierart des Kettengleitmittels. Bei Betrieben, die die Bandschmierlösung kontinuierlich applizieren, kann die Versorgung der Leergutüberduschung an beliebiger Stelle abgezweigt werden, bei alternierendem Puls-Pause-Betrieb muß vor dem Taktventil die Lösung in einer separaten Leitung zur Überduschungsstation geführt werden. In diesem Fall muß der Düsenstock mit einem separaten Magnetventil versehen werden, das mit dem Bandvortrieb synchron geschaltet ist. Im Fall der kontinuierlichen Kettengleitmitteldosage kann dieses zusätzliche Ventil entfallen. Im Vergleich zum Stand der Technik sind die Kosten für eine solche Modifikation sehr gering bei deutlicher Verbesserung des Hygienestandards.

Die Vorteile dieses neuen Systems zur Leergutüberduschung mit einem bekannten Kettengleitmittel liegen auf der Hand:
- sehr geringer zusätzlicher apparativer Aufwand;
   - zusätzliche Düsenstöcke mit Zuleitung bei Dauerdosage des Schmiermittels;
- Verbesserung der Arbeitssicherheit;
   - die Kettengleitmittellösung wird üblicherweise automatisch über eine mengenproportionale Dosierstation im gesamten Flaschenkeller an die Verbrauchsorte verteilt. Es entfällt daher das handling von Kanistern, da die Kettengleitmittel in Fässern, Containern oder Tankzügen angeliefert und automatisch ohne manuellen Kontakt dosiert werden;
   - keine Verwechslungsgefahr, wenn ein Produkt für verschiedene Anwendungen verwendet wird;
   - die verwendeten Produkte sind ökologisch besonders vorteilhaft und zeichnen sich inbesondere durch geringe Abwasserbelastung aus: pH-neutral, P-frei, nitratfrei, komplexierungsmittelfrei, geringer CSB-Wert (chemischer Sauerstoff-Bedarf) (280 mg/g), keine Lösevermittler, geringe Wassergefährdung, da Wassergefährdungsklasse 1 = wenig wassergefährdend;
- zusätzliche anwendungstechnische Vorteile:
   - Abbau von Verunreinigung im Sprüh- und Verteilsystem;
   - geeignet für alle bekannten Materialien, wie Glas, Kunststoff (PET, PC, PVC, PE, PP, etc.) und Metalle;
   - hohe Reinigungsaktivität, Schleimbildner werden aktiv abgebaut;
   - CO₂-unabhängig, daher besonders für kohlensaure Getränke geeignet;
   - Wirksamkeit weitgehend pH-Wert-unabhängig;
   - wasserqualitätsunabhängig, sowohl wasserhärte- als auch anionenunabhängig;
   - für harte, weiche, enthärtete Wässer geeignet, Wassertemperatur-unabhängig;
   - Produkte sind schaumfrei, aktiv korrosionsinhibierend, geruchlos, Konzentration einfach bestimmbar und damit universell einsetzbar;
- die durch dieses erfindungsgemäße Verfahren erzielte Verbesserung des Hygiene-Standards steht in einem sehr positiven Verhältnis zum apparativen und damit finanziellen Aufwand.

Insgesamt bietet die vorliegende Erfindung einen Vorteil bei der Vermeidung sekundärer Reinfektionsquellen. Die Anwendung des Verfahrens im Flaschenkeller wurde dabei nur beispielhaft am Beispiel Leergutüberduschung im Flaschenkeller eines Getränkgeabfüllbetriebs gewählt, da dort zur Zeit der Marktbedarf und die Reinfektionsgefahr am größten sind. Nach gleicher Bauart können aber auch alle anderen sekundären Reinfektionsquellen preiswert und erfolgreich nach diesem erfindungsgemäßen Verfahren eliminiert werden. Beispielhafte Anwendungen sind: Leergutüberduschung im Flaschen- und Faßkeller, Leergutüberduschung KEG- und Container.

Ein weiteres bevorzugtes Anwendungsgebiet des erfindungsgemäßen Verfahrens ist die Außenreinigung und Desinfektion über ein zentrales Niederdruckschaumsystem. In Nahrungsmittelbetrieben werden immer häufiger Niederdruckschaumsysteme zur Außenreinigung von Apparaten und Maschinen verwendet. Dabei werden sowohl mobile als auch zentrale Dosierstationen mit Satelliten verwendet. Die zentralen Schaumsysteme sind teilweise mit fest installierten, automatischen Systemen ausgestattet, die nach Produktionsablauf oder in Pausen automatisch eine Außenreinigung der Maschinen und Anlagen in Gang setzen. Dabei wird ein komplettes Hygieneprogramm durchlaufen. Da in Abfüllbetrieben der Getränkeindustrie, Milchwirtschaft u.ä. Transportbänder mit zentraler "Schmiermittelversorgung" vorhanden sind, kann das erfindungsgemäße Verfahren auch in solchen Schaumanlagen verwendet werden. Zur Verschäumung muß bei Bedarf die Luftansaugöffnung der Geräte geöffnet werden oder ein spezieller Schaumverstärker zugesetzt werden. Derart ausgestattet kann der gesamte Abfüll- und Verpackungsbereich mit lediglich einem Hygieneprodukt ausgestattet und betrieben werden. Die Einsparungen und Verbesserungen der Hygiene- und Arbeitssicherheit sind sehr groß.

Die erfindungsgemäß einzusetzenden Reinigungs- und Desinfektionsmittel auf Basis von Aminen und gegebenenfalls üblichen Verdünnungsmitteln oder Hilfs- bzw. Zusatzstoffen enthalten mindestens ein sekundäres und/oder tertiäres Amin der Formeln (Ia), (Ib), (IIa), (IIb), (IIc), (IIIa) oder (IIIb) und/oder ein Salz derartiger Amine, wobei der Anteil der Amine an der Gesamtformulierung 1 bis 100 Gew.-% beträgt. Sie sind aus der älteren deutschen Patentanmeldung DE-A-39 05 548 als Kettengleitmittel bekannt.

Gemäß einer Ausführungsform der vorliegenden Erfindung wird als Reinigungs- und Desinfektionsmittel mindestens ein sekundäres Amin der allgemeinen Formeln (Ia) oder (Ib)

R-NH-R¹ (Ia)

R-N⁺H₂-R¹ X⁻ (Ib)

eingesetzt, wobei die Reste R und R¹ jeweils unabhängig voneinander bedeuten:
- einen substituierten oder unsubstituierten, linearen oder verzweigten, gesättigten oder einfach oder mehrfach ungesättigten Alkylrest mit 6 bis 22 C-Atomen, der als Substituenten mindestens einen Amin-, Imin-, Hydroxy-, Halogen- und/oder Carboxyrest aufweisen kann,
- einen substituierten oder unsubstituierten Phenylrest, der als Substituenten mindestens einen Amin-, Imin-, Hydroxy-, Halogen-, Carboxy- und/oder einen linearen oder verzweigten, gesättigten oder einfach oder mehrfach ungesättigten Alkylrest mit 6 bis 22 C-Atomen aufweisen kann,
   und X⁻ ein Anion aus der Gruppe Amidosulfonat, Nitrat, Halogenid, Sulfat, Hydrogencarbonat, Carbonat, Phosphat oder

   R²-COO⁻

bedeutet, wobei der Rest R² für
- Wasserstoff,
- einen substituierten oder unsubstituierten, linearen oder verzweigten Alkylrest mit 1 bis 20 C-Atomen oder Alkenylrest mit 2 bis 20 C-Atomen, die als Substituenten mindestens einen Hydroxy-, Amin- oder Iminrest aufweisen können, oder
- einen substituierten oder unsubstituierten Phenylrest, der als Substituenten einen Alkylrest mit 1 bis 20 C-Atomen aufweisen kann,
steht.

In den vorstehend genannten allgemeinen Formeln (Ia) und (Ib) kommen als Substituenten R und R¹ somit die folgenden Reste in Frage: n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Uneicosyl und n-Docosyl sowie die verzweigtkettigen Isomere der genannten Alkylreste. Anstelle der gesättigten Alkylreste können R und R¹ auch die entsprechenden - einfach oder mehrfach - ungesättigten Alkylreste bedeuten, die gleichfalls linear oder verzweigt sein können. Die vorstehend angeführten Reste können auch substituiert sein, wobei als Substituenten eine oder mehrere Amin-, Imin-, Hydroxy-, Halogen- oder Carboxygruppen in Frage kommen. Darüber hinaus können die Reste R und R¹ auch Phenylreste bedeuten, die gleichfalls mit einer oder mehreren Amin-, Imin-, Hydroxy-, Halogen- oder Carboxygruppen substituiert sein können. Auch Alkylphenylreste kommen für R und R¹ in Frage, wobei der Alkylrest 6 bis 22 C-Atome enthalten und gleichfalls linear oder verzweigt, gesättigt oder einfach oder mehrfach ungesättigt sein kann. Als Halogensubstituenten sind in allen Fällen Chlor oder Brom bevorzugt.

Als Beispiele für sekundäre Amine vom Typ der allgemeinen Formel (Ia), die im Rahmen der vorliegenden Erfindung eingesetzt werden, seien genannt: Dicocosfettalkyl-amin, Distearyl-amin und Ditalgfettalkyl-amin.

Als Anion X⁻ kommen - neben den bereits angeführten anorganischen Anionen - auch Anionen organischer Säuren vom Typ R²-COO⁻ in Frage. Der Rest R² kann hierbei auch Wasserstoff und niedere Alkyl- bzw. Alkenylreste bedeuten; im übrigen gelten hierfür die vorstehenden Erläuterungen zu R und R¹ in analoger Weise.

Als Beispiele für organische Anionen X⁻ vom Typ R²-COO⁻ seien genannt: Formiat, Acetat, Oleat, Glycolat, Lactat, Gluconat, Benzoat und Salicylat.

Als Beispiele für sekundäre Amine vom Typ der allgemeinen Formel (Ib), die gleichfalls im Rahmen der vorliegenden Erfindung bevorzugt eingesetzt werden, kommen die vorstehend angeführten Verbindungen vom Typ der allgemeinen Formel (Ia) in Form ihrer Salze mit den zuvor genannten organischen Anionen in Frage.

Gemäß einer weiteren Ausführungsform der vorliegenden Erfindung enthält das einzusetzende Reinigungs- und Desinfektionsmittel mindestens ein sekundäres Diamin der allgemeinen Formeln (IIa), (IIb) oder (IIc)

R-NH-(CH₂)₃NH₂ (IIa)

R-NH-(CH₂)₃N⁺H₃ X⁻ (IIb)

R-N⁺H₂-(CH₂)₃-N⁺H₃ 2X⁻ (IIc)

wobei die Reste R und X⁻ jeweils die vorstehend für die allgemeinen Formeln (Ia) und (Ib) angegebenen Bedeutungen haben.

Als Beispiele für sekundäre Diamine vom Typ der allgemeinen Formeln (IIa), (IIb) und (IIc), die im Rahmen der vorliegenden Erfindung bevorzugt sind, seien genannt: N-Lauryl-propylendiamin und N-Talgfettalkyl-propylendiamin, jeweils in Form der freien Amine sowie in Form der Acetat-Salze.

Gemäß einer dritten Ausführungsform der vorliegenden Erfindung wird mindestens ein tertiäres Amin der allgemeinen Formeln (IIIa) oder (IIIb)

R-NR³R⁴ (IIIa)

R-N⁺HR³R⁴ X⁻ (IIIb)

eingesetzt, wobei die Reste R und X⁻ jeweils die für die allgemeinen Formeln (Ia) und (Ib) angegebenen Bedeutungen haben und die Reste R³ und R⁴ jeweils unabhängig voneinander bedeuten:
- einen substituierten oder unsubstituierten, linearen oder verzweigten Alkylrest mit 1 bis 20 C-Atomen oder Alkenylrest mit 2 bis 20 C-Atomen, die als Substituenten mindestens einen Hydroxy-, Amin- oder Iminrest aufweisen können, oder
- einen substituierten oder unsubstituierten Phenylrest, der als Substituenten einen Alkylrest mit 1 bis 20 C-Atomen aufweisen kann.

Für die Reste R³ und R⁴ gelten wieder sinngemäß diejenigen Erläuterungen, die vorstehend im Zusammenhang mit den Resten R und R¹ gemacht worden sind.

Als Beispiele für tertiäre Amine vom Typ der allgemeinen Formeln (IIIa) und (IIIb), die im Rahmen der vorliegenden Erfindung bevorzugt sind, seien genannt: N,N-Dipropyl-N-laurylamin und das entsprechende Acetat-Salz.

Im Rahmen dieser dritten Ausführungsform ist es ferner bevorzugt, daß mindestens ein tertiäres Amin der allgemeinen Formeln (IVa) oder (IVb)

R-N(CH₃)₂ (IVa)

R-N⁺H(CH₃)₂ X⁻ (IVb)

eingesetzt wird, wobei die Reste R und X⁻ jeweils die für die allgemeinen Formeln (Ia) und (Ib) angegebenen Bedeutungen haben.

Als Beispiele für tertiäre Amine vom Typ der allgemeinen Formeln (IVa) und (IVb), die im Rahmen der Erfindung gleichfalls bevorzugt sind, seien genannt: N,N-Dimethyl-N-laurylamin, N,N-Dimethyl-N-hexadecylamin, N,N-Dimethyl-N-cocosfettalkylamin, N,N-Dimethyl-N-cetylamin sowie die entsprechenden Acetat-Salze.

Sekundäre und tertiäre Amine, die den vorstehend angegebenen allgemeinen Formeln entsprechen, können nach literaturbekannten Verfahren hergestellt werden und werden im übrigen auch zum Teil als Handelsprodukte angeboten, beispielsweise von der Firma Hoechst AG, Frankfurt am Main, Deutschland, unter der Bezeichnung GENAMIN^{R} oder von der Firma Lonza, Basel, Schweiz, unter der Bezeichnung LONZABAC^{R} 12.

Nach einer bevorzugten Ausführungsform der vorliegenden Erfindung werden sekundäre und/oder tertiäre Amine der vorstehend angeführten allgemeinen Formeln (Ia) bis (IVb) eingesetzt, wobei die Reste R, R¹, R², R³, R⁴ und X⁻ die folgenden Bedeutungen aufweisen:
- R und R¹ stehen jeweils unabhängig voneinander für einen linearen oder verzweigten, gesättigten oder einfach oder mehrfach ungesättigten Alkylrest mit 12 bis 18 C-Atomen,
- R³ und R⁴ stehen jeweils unabhängig voneinander für einen linearen oder verzweigten Alkylrest mit 1 bis 6 C-Atomen oder Alkenylrest mit 2 bis 6 C-Atomen,
- X⁻ steht für den Rest R²-COO⁻, wobei R² Wasserstoff, CH₃-, HO-CH₂- oder CH₃-CH(OH)- bedeutet.

Im Sinne der Erfindung sind ferner solche Mittel bevorzugt, die
5 bis 40 Gew.-%, insbesondere 10 bis 20 Gew.-%, an sekundären und/oder tertiären Aminen und/oder Salzen derartiger Amine sowie
95 bis 60 Gew.-%, insbesondere 90 bis 80 Gew.-%, Wasser als Verdünnungsmittel und gegebenenfalls Hilfs- bzw. Zusatzstoffe,
jeweils bezogen auf die Gesamtformulierung, enthalten.

Die einzusetzenden Mittel zeigen in Form ihrer wäßrigen Lösungen entweder Klarlöslichkeit oder Opaleszenz.

Als Hilfs- und/oder Zusatzstoffe im Sinne der vorliegenden Erfindung kommen insbesondere Lösungsvermittler in Betracht, beispielsweise Alkohole, Polyalkohole, Ether oder Polyether, insbesondere Isopropanol, Butylglykol, Butyldiglykol oder Ethylenglykolether. Die Menge des zu verwendenden Lösungsvermittlers richtet sich im Einzelfall nach dem eingesetzten Amin, der Fachmann wird im Einzelfall die erforderliche Menge an Lösungsvermittler durch Ausprobieren ermitteln. Im allgemeinen sind Zusätze an Lösungsvermittler im Bereich von 5 bis 20 Gew.-%, bezogen auf die Gesamtformulierung, hinreichend.

Als Hilfs- und/oder Zusatzstoffe im Sinne der vorliegenden Erfindung kommen ferner insbesondere nichtionische und/oder anionische Tenside in Betracht, beispielsweise alkoxylierte Fettamine, Fettalkohole, alkoxylierte Fettalkohole und in hydrophilen Lösungsmitteln lösliche Alkylbenzolsulfonate. Im allgemeinen sind Tensid-Zusätze im Bereich von 5 bis 10 Gew.-%, bezogen auf die Gesamtformulierung, hierfür ausreichend.

Die erfindungsgemäß einzusetzenden Mittel weisen vorzugsweise einen pH-Wert im Bereich von 4 bis 11, insbesondere im Bereich von 5 bis 8, auf. Sofern der pH-Wert nicht bereits in diesem Bereich liegt, kann er durch Zugabe einer Säure, vorzugsweise einer Säure mit dem vorstehend definierten Anion X⁻, beispielsweise mit Essigsäure oder Ameisensäure, auf den gewünschten Wert eingestellt werden.

Im Sinne der Erfindung ist es ferner bevorzugt, daß die Mittel eine dynamische Viskosität von weniger als 300 mPa.s, insbesondere von weniger als 150 mPa.s und besonders bevorzugt im Bereich von 20 bis 100 mPa.s - jeweils bei 20 °C - aufweisen. Eine gesonderte Einstellung der Viskosität auf die genannten Werte ist im allgemeinen nicht erforderlich bzw. erfolgt gegebenenfalls durch Zusatz geeigneter Mengen des Verdünnungsmittels Wasser oder eines Lösungsvermittlers.

Die erfindungsgemäß einzusetzenden Mittel lassen sich durch einfaches Vermischen der Aminkomponenten, gegebenenfalls unter Zusatz von Wasser und der genannten Hilfs- bzw. Zusatzstoffe, herstellen.

Gemäß einer besonders bevorzugten Ausführungsform der vorliegenden Erfindung enthalten die einzusetzenden Mittel:
a) 2 bis 10 Gew.-% eines sekundären Amins der allgemeinen Formeln (IIb) und/oder (IIc),
b) 2 bis 10 Gew.-% eines tertiären Amins der allgemeinen Formeln (IIIb) und/oder (IV)b,
c) Rest: Wasser und gegebenenfalls Hilfs- bzw. Zusatzstoffe,
wobei die Komponenten a und b im Gewichtsverhältnis von a zu b wie 1 : 2 bis 3 : 1, vorzugsweise 2 : 1, vorliegen.

Insbesondere betrifft die vorliegende Erfindung die Verwendung der vorstehend beschriebenen Mittel in Form einer 5 bis 40 Gew.-%igen, vorzugsweise 1 bis 10 Gew.-%igen, wäßrigen Lösung als Reinigungs- und Desinfektionsmittel zur Überduschung von Leergut in der Lebensmittel- und Getränkeindustrie.

Die erfindungsgemäß einzusetzenden Produkte verursachen keine Spannungsrißkorrosion und können daher für PET und PC-Gebinde problemlos eingesetzt werden. (PET = Polyethylenterephthalat, PC= Polycarbonat). Besonders bevorzugt für klar-wasserlösliche Konzentrate sind pH-Werte < 8,5.

### Ausführungsbeispiel

Die nachfolgenden Testergebnisse zeigen die Wirkung der erfindungsgemäß einzusetzenden Reinigungs- und Desinfektionsmittel bei einer Reihe von Mikroorganismen, die in der Lebensmittel- und Getränkeindustrie häufig vorkommen.

### Versuchsbedingungen

Das Mittel wurde in den Einsatzkonzentrationen von 0,15 und 0,3 % bei Einwirkungszeiten von 10 Minuten im Suspensionstest gegenüber getränkeschädlichen Bakterien und Hefen getestet. Die Einwirkungstemperaturen lagen entsprechend der Praxisverhältnisse bei 10 °C und 20 °C. Bei den Versuchen bei 10 °C wurde zusätzlich eine organische Belastung (3 % Zusatz einer 10 %igen Bierwürze) berücksichtigt. Bei dieser Temperatur wurden die Teststämme in der lag-Phase eingesetzt, während die Versuche mit Einwirkungstemperaturen von 20 °C mit logarithmisch wachsenden Zellen durchgeführt wurden. Die Ausgangskeimzahlen wurden auf 10⁶ bis 10⁸ Zellen pro Milliliter eingestellt. Zur genauen Ermittlung der Keimzahlen wurden Verdünnungsreihen mittels NBB- bzw. OFS-Agar-Gußplatten bzw. entsprechende Membranfilterkulturen angesetzt. Der Nachweis auf überlebende Keime erfolgte duch Membranfiltration der gesamten Testlösung nach erfolgter Desinfektionsmittelinaktivierung.

Eingesetzt wurde ein 15 Gew.-%iges wäßriges Konzentrat von Laurylpropylendiammoniumacetat, das entsprechend verdünnt wurde. Das Schema des Suspensionstests gibt die Versuchsdurchführung wieder (Seite 32).

### Bedeutung der Testorganismen

Bei den **Hefen** wurden sowohl bierschädliche, als auch limonadenschädliche Teststämme berücksichtigt. Dabei handelt es sich ausschließlich um Originalisolate aus der Praxis. Diese Stämme hatten sich bei früheren Versuchen meist als sehr resistent gegenüber Desinfektionsmaßnahmen erwiesen. Die Ergebnisse können daher als repräsentativ auch für den Abtötungserfolg gegenüber anderen, in der Getränkeindustrie auftretenden Hefearten angesehen werden.

Als bierschädliche Art wurde Saccharomyces cerevisiae subsp. diastaticus überprüft. Der vorliegende Stamm neigt relativ stark zur Ausbildung von Ascosporen, die höhere Resistenzen aufweisen als die vegetativen Zellen. Diese übervergärende Hefe ist wesentlich widerstandsfähiger als Kulturhefen und vergleichbar mit anderen in der Brauerei auftretenden Fremdhefen.

Als typische Schädlinge in der AFG-Branche wurden die drei Arten Saccharomyces cerevisiae, Zygosaccharomyces bailii und Hansenula anomala überprüft.

S. cerevisiae ist eine säuretolerante, fruchtspezifische Hefe, die sowohl Limonaden als auch Fruchtsäfte verderben kann. Sie bildet Ascosporen und kommt sehr häufig im Milieu der Getränkeherstellung vor.

Z. bailii ist ebenfalls sehr säuretolerant und ist zusätzlich auch sehr unempfindlich gegenüber Konservierungsstoffen (Benzoesäure, Sorbinsäure, Ameisensäure). Die Art zeichnet sich noch zusätzlich durch eine hohe Osmotoleranz aus, so daß auch Infektionen in Fruchtkonzentraten und Zuckersirupen möglich sind. Z. bailii bildet ebenfalls Ascosporen und gehört zu den widerstandsfähigsten Hefen der Getränkeindustrie. Die Art ist vor allem in der Fruchsaftbranche von Bedeutung und wirkt sich bei Infektionen ähnlich aus wie S. cerevisiae.

Bei den beiden Arten werden also nicht nur Geschmacks- und Geruchsfehler hervorgerufen, sondern auch hohe CO₂-Drucke bis hin zu Bombagen sowie Trübungsinstabilitäten infolge von pektolytischer Aktivität.

Hansenula anomala ist sowohl in der Brauerei als auch in der AFG-Branche ein häufiger Kontaminant. Diese Art tritt aber nur in stillen Getränken als Schädling in Erscheinung, so z.B. in Fruchtsäften, wo durch die Bildung von Amylacetat und anderen unerwünsachten Stoffwechselprodukten äußerst unangenehme Geruchs- und Geschmacksfehler hervorgerufen werden. H. anomala bildet ebenfalls Ascosporen und ist bei Desinfektionsmaßnahmen repräsentativ für die Abtötung von Kahmhefen.

Bei den **Bakterien** wurden die drei obligat bierschädlichen Arten Lactobacillus lindneri, Lactobacillus frigidus sowie Pedioccus damnosus getestet. Besonders die beiden Laktobazillen sind sehr widerstandsfähig gegenüber Desinfektionsmaßnahmen. L. lindneri weist eine hohe Resistenz gegenüber Desinfektionsmitteln auf. L. frigidus bildet Schleimkapseln und ist dadurch insgesamt besser geschützt. Diese drei Arten sind repräsentativ für die Abtötungseffizienz bei allen bierschädlichen Bakterien.

Als Vertreter der Gram-negativen Begleitflora wurde schließlich noch Enterobacter cloacae getestet. Diese Art bildet ebenfalls Schleimkapseln und gehört deshalb zu den resistentesten Enterobacteriaceen und ist deshalb auch als Maßstab für die Abtötung von Escherichia coli, Coliformen Bakterien und anderen Fäkalindikatoren zu bewerten.

### Wirkung bei brauereispezifischen Mikroorganismen

Das Mittel zeigte sowohl bei bierschädlichen Hefen als auch bei bierschädlichen Bakterien und Enterobacter cloacae eine ausgezeichnete Wirkung. So konnte bei einer Gebrauchskonzentration von 0,3 % auch bei der kürzesten Einwirkungszeit von 10 Minuten bei allen Testorganismen ein 100 %iger Abtötungserfolg erzielt werden. Auch bei einer Konzentration von 0,15 % wurden die Keime quantitativ abgetötet.

Diese Ergebnisse wurden sowohl bei lag- als auch bei log-Phase-Kulturen erzielt und gelten auch für die Einwirkungstemperaturen von 10 und 20 °C. Auch eine organische Belastung von 3 % Würze in den Testlösungen beeinträchtigte den Abtötungserfolg nicht.

### Wirkung bei Schädlingen der Fruchtsaftindustrie

Auch gegenüber getränkeschädlichen Hefen der AFG-Branche und Fruchtsaftindustrie wurde ein 100 %iger Abtötungserfolg erzielt. Diese Ergebnisse gelten sowohl für die bevorzugte Konzentration von 0,3 % als auch für die Konzentration von 0,15 %. Eine quantitative Abtötung wurde sowohl bei Kulturen der lag- als auch der log-Phase erzielt. Durch niedrigere Einwirkungstemperaturen von 10 °C und organische Belastung des Testsubstrates wurden keine Beeinträchtigungen der Effizienz festgestellt.

### Bewertung der Ergebnisse

Das getestete Desinfektionsmittel zeigte unter allen Testbedingungen und gegenüber allen getränkeschädlichen Testorganismen (Brauerei und Getränkeindustrie) einen 100 %igen Abtötungseffekt.

| Versuch | 1 | 2 |
|---|---|---|
| Keim: | S. diastaticus | S. diastaticus |
| Wachstumsphase/physiol. Zustand: | log | lag |
| Einwirkungstemperatur: | 20 °C | 10 °C (+ org. Belastg.) |
| Ausgangskeimzahl/ml: | 3,0 . 10⁷ | 1,3 . 10⁶ |

| Konzentration: 0,15 % | | |
|---|---|---|
| Einwirkzeit 10 min: | 0 | 0 |
| Einwirkzeit 20 min: | 0 | 0 |

| Konzentration: 0,3 % | | |
|---|---|---|
| Einwirkzeit 10 min: | 0 | 0 |
| Einwirkzeit 20 min: | 0 | 0 |
| Keimreduzierung (%): | 100 | 100 |

| Versuch | 3 | 4 |
|---|---|---|
| Keim: | Z. bailii | Z. bailii |
| Wachstumsphase/physiol. Zustand: | log | lag |
| Einwirkungstemperatur: | 20 °C | 10 °C (+ org. Belastg.) |
| Ausgangskeimzahl/ml: | 5,0 . 10⁶ | 2,4 . 10⁶ |

| Konzentration: 0,15 % | | |
|---|---|---|
| Einwirkzeit 10 min: | 0 | 2 |
| Einwirkzeit 20 min: | 0 | 0 |

| Konzentration: 0,3 % | | |
|---|---|---|
| Einwirkzeit 10 min: | 0 | 0 |
| Einwirkzeit 20 min: | 0 | 0 |
| Keimreduzierung (%): | 100 | 100 |

| Versuch | 5 | 6 |
|---|---|---|
| Keim: | L. lindneri | L. lindneri |
| Wachstumsphase/physiol. Zustand: | log | lag |
| Einwirkungstemperatur: | 20 °C | 10 °C (+ org. Belastg.) |
| Ausgangskeimzahl/ml: | 3,6 . 10⁶ | 7,0 . 10⁶ |

| Konzentration: 0,15 % | | |
|---|---|---|
| Einwirkzeit 10 min: | 0 | 0 |
| Einwirkzeit 20 min: | 0 | 0 |

| Konzentration: 0,3 % | | |
|---|---|---|
| Einwirkzeit 10 min: | 0 | 0 |
| Einwirkzeit 20 min: | 0 | 0 |
| Keimreduzierung (%): | 100 | 100 |

| Versuch | 7 | 8 |
|---|---|---|
| Keim: | P. damnosus | P. damnosus |
| Wachstumsphase/physiol. Zustand: | log | lag |
| Einwirkungstemperatur: | 20 °C | 10 °C (+ org. Belastg.) |
| Ausgangskeimzahl/ml: | 6,5 . 10⁷ | 2,5 . 10⁷ |

| Konzentration: 0,15 % | | |
|---|---|---|
| Einwirkzeit 10 min: | 0 | 0 |
| Einwirkzeit 20 min: | 0 | 0 |

| Konzentration: 0,3 % | | |
|---|---|---|
| Einwirkzeit 10 min: | 0 | 0 |
| Einwirkzeit 20 min: | 0 | 0 |
| Keimreduzierung (%): | 100 | 100 |

| Versuch | 9 | 10 |
|---|---|---|
| Keim: | F. cloacae | F. cloacae |
| Wachstumsphase/physiol. Zustand: | log | lag |
| Einwirkungstemperatur: | 20 °C | 10 °C (+ org. Belastg.) |
| Ausgangskeimzahl/ml: | 7,0 . 10⁶ | 5,0 . 10⁶ |

| Konzentration: 0,15 % | | |
|---|---|---|
| Einwirkzeit 10 min: | 0 | 0 |
| Einwirkzeit 20 min: | 0 | 0 |

| Konzentration: 0,3 % | | |
|---|---|---|
| Einwirkzeit 10 min: | 0 | 0 |
| Einwirkzeit 20 min: | 0 | 0 |
| Keimreduzierung (%): | 100 | 100 |

| Versuch | 11 | 12 |
|---|---|---|
| Keim: | L. frigidus | L. frigidus |
| Wachstumsphase/physiol. Zustand: | log | lag |
| Einwirkungstemperatur: | 20 °C | 10 °C (+ org. Belastg.) |
| Ausgangskeimzahl/ml: | 3,0 . 10⁶ | 2,6 . 10⁷ |

| Konzentration: 0,15 % | | |
|---|---|---|
| Einwirkzeit 10 min: | 0 | 0 |
| Einwirkzeit 20 min: | 0 | 0 |

| Konzentration: 0,3 % | | |
|---|---|---|
| Einwirkzeit 10 min: | 0 | 0 |
| Einwirkzeit 20 min: | 0 | 0 |
| Keimreduzierung (%): | 100 | 100 |

| Versuch | 13 | 14 |
|---|---|---|
| Keim: | H. anomala | H. anomala |
| Wachstumsphase/physiol. Zustand: | log | lag |
| Einwirkungstemperatur: | 20 °C | 10 °C (+ org. Belastg.) |
| Ausgangskeimzahl/ml: | 1,4 . 10⁸ | 2,0 . 10⁷ |

| Konzentration: 0,15 % | | |
|---|---|---|
| Einwirkzeit 10 min: | 0 | 0 |
| Einwirkzeit 20 min: | 0 | 0 |

| Konzentration: 0,3 % | | |
|---|---|---|
| Einwirkzeit 10 min: | 0 | 0 |
| Einwirkzeit 20 min: | 0 | 0 |
| Keimreduzierung (%): | 100 | 100 |

| Versuch | 15 | 16 |
|---|---|---|
| Keim: | S. cerevisiae | S. cerevisiae |
| Wachstumsphase/physiol. Zustand: | log | lag |
| Einwirkungstemperatur: | 20 °C | 10 °C (+ org. Belastg.) |
| Ausgangskeimzahl/ml: | 8,8 . 10⁶ | 2,5 . 10⁷ |

| Konzentration: 0,15 % | | |
|---|---|---|
| Einwirkzeit 10 min: | 0 | 0 |
| Einwirkzeit 20 min: | 0 | 0 |

| Konzentration: 0,3 % | | |
|---|---|---|
| Einwirkzeit 10 min: | 0 | 0 |
| Einwirkzeit 20 min: | 0 | 0 |
| Keimreduzierung (%): | 100 | 100 |

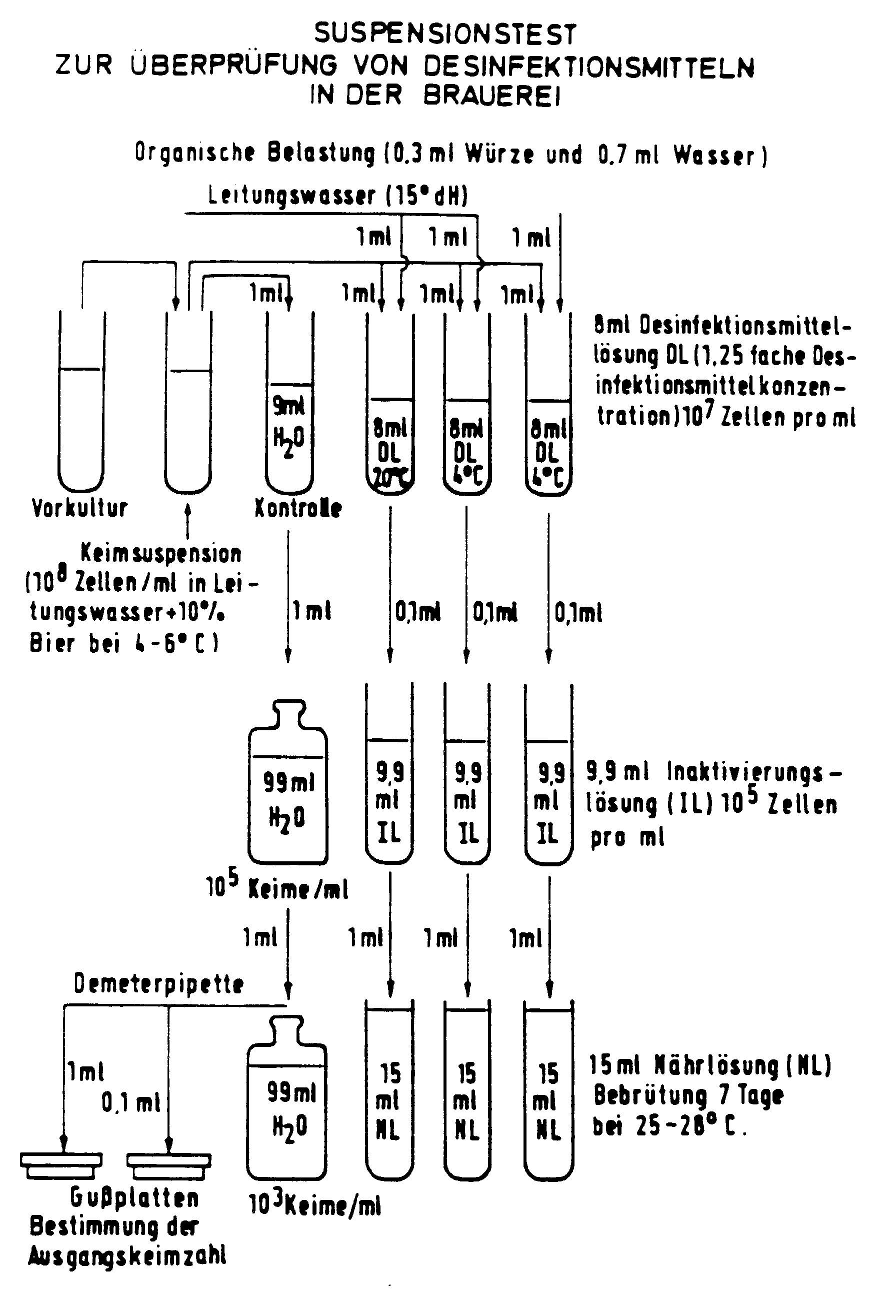

## Patentansprüche

1. Verfahren zur Desinfektion und Reinigung in der Lebensmittel- und Getränkeindustrie unter Verwendung von Aminen, dadurch gekennzeichnet, daß man ein an sich bekanntes Gleitmittel auf Basis von sekundären und/oder tertiären Aminen und/oder Salzen derartiger Amine und gegebenenfalls üblichen Verdünnungsmitteln oder Hilfs- bzw. Zusatzstoffen zur Überduschung von Leergut verwendet, welches
(a) mindestens ein sekundäres Amin der allgemeinen Formeln (Ia) oder (Ib)
R-NH-R¹ (Ia)
R-N⁺H₂-R¹ X⁻ (Ib)
und/oder
(b) mindestens ein sekundäres Diamin der allgemeinen Formeln (IIa), (IIb) oder (IIc)
R-NH-(CH₂)₃NH₂ (IIa)
R-NH-(CH₂)₃N⁺H₃ X⁻ (IIb)
R-N⁺H₂-(CH₂)₃-N⁺H₃ 2X⁻ (IIc)
und/oder
(c) mindestens ein tertiäres Amin der allgemeinen Formeln (IIIa) oder (IIIb)
R-NR³R⁴ (IIIa)
R-N⁺HR³R⁴ X⁻ (IIIb)
enthält, wobei die Reste R und R¹ jeweils unabhängig voneinander bedeuten:
- einen substituierten oder unsubstituierten, linearen oder verzweigten, gesättigten oder einfach oder mehrfach ungesättigten Alkylrest mit 6 bis 22 C-Atomen, der als Substituenten mindestens einen Amin-, Imin-, Hydroxy-, Halogen- und/oder Carboxyrest aufweisen kann,
- einen substituierten oder unsubstituierten Phenylrest, der als Substituenten mindestens einen Amin-, Imin-, Hydroxy-, Halogen-, Carboxy- und/oder einen linearen oder verzweigten, gesättigten oder einfach oder mehrfach ungesättigten Alkylrest mit 6 bis 22 C-Atomen aufweisen kann,
und X⁻ ein Anion aus der Gruppe Amidosulfonat, Nitrat, Halogenid, Sulfat, Hydrogencarbonat, Carbonat, Phosphat oder
R²-COO⁻
bedeutet, wobei der Rest R² für
- Wasserstoff,
- einen substituierten oder unsubstituierten, linearen oder verzweigten Alkylrest mit 1 bis 20 C-Atomen oder Alkenylrest mit 2 bis 20 C-Atomen, die als Substituenten mindestens einen Hydroxy-, Amin- oder Iminrest aufweisen können, oder
- einen substituierten oder unsubstituierten Phenylrest, der als Substituenten einen Alkylrest mit 1 bis 20 C-Atomen aufweisen kann,
steht, und wobei die Reste R³ und R⁴ jeweils unabhängig voneinander bedeuten:
- einen substituierten oder unsubstituierten, linearen oder verzweigten Alkylrest mit 1 bis 20 C-Atomen oder Alkenylrest mit 2 bis 20 C-Atomen, die als Substituenten mindestens einen Hydroxy-, Amin- oder Iminrest aufweisen können, oder
- einen substituierten oder unsubstituierten Phenylrest, der als Substituenten einen Alkylrest mit 1 bis 20 C-Atomen aufweisen kann,
und wobei der Anteil der Amine an der Gesamtformulierung 1 bis 100 Gew.-% beträgt.

2. Verfahren nach Anspruch 1, dadurch gekennzeichnet, daß die Reste R, R¹, R², R³, R⁴ und X⁻ in den allgemeinen Formeln die folgenden Bedeutungen aufweisen:
- R und R¹ stehen jeweils unabhängig voneinander für einen linearen oder verzweigten, gesättigten oder einfach oder mehrfach ungesättigten Alkylrest mit 12 bis 18 C-Atomen,
- R³ und R⁴ stehen jeweils unabhängig voneinander für einen linearen oder verzweigten Alkylrest mit 1 bis 6 C-Atomen oder Alkenylrest mit 2 bis 6 C-Atomen,
- X⁻ steht für den Rest R²-COO⁻, wobei R² Wasserstoff, CH₃-, HO-CH₂- oder CH₃-CH(OH)- bedeutet.

3. Verfahren nach einem der Ansprüche 1 und 2, dadurch gekennzeichnet, daß man mindestens ein tertiäres Amin der allgemeinen Formeln (IVa) oder (IVb)
R-N(CH₃)₂ (IVa)
R-N⁺H(CH₃)₂ X⁻ (IVb)
einsetzt, wobei die Reste R und X⁻ jeweils die für die allgemeinen Formeln (Ia) und (Ib) angegebenen Bedeutungen haben.

4. Verfahren nach einem der Ansprüche 1 bis 3, dadurch gekennzeichnet, daß man
5 bis 40 Gew.-%, insbesondere 10 bis 20 Gew.-%, an Aminen und
95 bis 60 Gew.-%, insbesondere 90 bis 80 Gew.-%, Wasser und/oder Hilfs- bzw. Zusatzstoffe,
jeweils bezogen auf die Gesamtformulierung, einsetzt.

5. Verfahren nach Anspruch 4, dadurch gekennzeichnet, daß man als Hilfs- bzw. Zusatzstoff Lösungsvermittler einsetzt.

6. Verfahren nach Anspruch 4, dadurch gekennzeichnet, daß man als Hilfs- bzw. Zusatzstoff nichtionische und/oder anionische Tenside einsetzt.

7. Verfahren nach einem der Ansprüche 1 bis 6, dadurch gekennzeichnet, daß man einen pH-Wert im Bereich von 4 bis 11, vorzugsweise im Bereich von 5 bis 8, einstellt.

8. Verfahren nach einem der Ansprüche 1 bis 6, dadurch gekennzeichnet, daß man eine dynamische Viskosität von weniger als 300 mPa.s, vorzugsweise im Bereich von 20 bis 100 mPa.s, einstellt.

9. Verfahren nach einem der Ansprüche 1 bis 8, dadurch gekennzeichnet, daß man
a) 2 bis 10 Gew.-% eines sekundären Amins der allgemeinen Formeln (IIb) und/oder (IIc),
b) 2 bis 10 Gew.-% eines tertiären Amins der allgemeinen Formeln (IIIb) und/oder (IV)b,
c) Rest: Wasser und gegebenenfalls Hilfs- bzw. Zusatzstoffe,
einsetzt, wobei die Komponenten a und b im Gewichtsverhältnis von a zu b wie 1 : 2 bis 3 : 1, vorzugsweise 2 : 1, vorliegen.

10. Verfahren nach einem der Ansprüche 1 bis 9, dadurch gekennzeichnet, daß man als Leergut Getränkeflaschen, insbesondere Glasflaschen oder Kunststoffflaschen, Dosen, Gläser, Fässer und/oder Getränkecontainer überduscht.

## Claims

1. A process for cleaning and disinfection in the food and beverage industry using amines, characterized in that a lubricant known **per se** based on secondary and/or tertiary amines and/or salts of such amines and, optionally, typical diluents or auxiliaries and additives is used for spraying empties, this lubricant containing
(a) at least one secondary amine corresponding to general formula (Ia) or (Ib)
R-NH-R¹ (Ia)
R-N⁺H₂-R¹ X⁻ (Ib)
and/or
(b) at least one secondary diamine corresponding to general formulae (IIa), (IIb) or (IIc)
R-NH-(CH₂)₃NH₂ (IIa)
R-NH-(CH₂)₃N⁺H₃ X⁻ (IIb)
R-N⁺H₂-(CH₂)₃-N⁺H₃ 2X⁻ (IIc)
and/or
(c) at least one tertiary amine corresponding to general formula (IIIa) or (IIIb)
R-NR³R⁴ (IIIa)
R-N⁺HR³R⁴ X⁻ (IIIb)
the substituents R and R¹ independently of one another having the following meanings:
- a substituted or unsubstituted, linear or branched, saturated or mono- or polyunsaturated alkyl radical containing 6 to 22 carbon atoms which may be substituted at least once by amine, imine, hydroxy, halogen and/or carboxy,
- a substitued or unsubstituted phenyl radical which may be substituted at least once by amine, imine, hydroxy, halogen, carboxy and/or a linear or branched, saturated or mono- or polyunsaturated alkyl radical containing 6 to 22 carbon atoms and
X⁻ is an anion from the group consisting of amidosulfonate, nitrate, halide, sulfate, hydrogen carbonate, carbonate, phosphate or
R²COO⁻
where R² is
- hydrogen,
- a substituted or unsubstituted, linear or branched alkyl radical containing 1 to 20 carbon atoms or an alkenyl radical containing 2 to 20 carbon atoms which may be substituted at least once by hydroxy, amine or imine or
- a substituted or unsubstituted phenyl radical which may be substituted by a C₁₋₂₀ alkyl radical,
and the substituents R³ and R⁴ independently of one another having the following meanings:
- a substituted or unsubstituted, linear or branched C₁₋₂₀ alkyl radical or C₂₋₂₀ alkenyl radical which may be substituted at least once by hydroxy, amine or imine or
- a substituted or unsubstituted phenyl radical which may be substituted by C₁₋₂₀ alkyl,
and the percentage content of the amines in the formulation as a whole being from 1 to 100% by weight.

2. A process as claimed in claim 1, characterized in that the substituents R, R¹, R², R³, R⁴ and X⁻ in the general formulae have the following meanings:
- R and R¹ independently of one another represent a linear or branched, saturated or mono- or polyunsaturated alkyl radical containing 12 to 18 carbon atoms,
- R³ and R⁴ independently of one another represent a linear or branched alkyl radical containing 1 to 6 carbon atoms or alkenyl radical containing 2 to 6 carbon atoms,
- X⁻ represents the group R²-COO⁻ where R² is hydrogen, CH₃-, HO-CH₂- or CH₃-CH(OH)-.

3. A process as claimed in claim 1 or 2, characterized in that at least one tertiary amine corresponding to general formula (IVa) or (IVb):
R-N(CH₃)₂ (IVa)
R-N⁺H(CH₃)₂ X⁻ (IVb)
is used, the substituents R and X⁻ having the meanings defined for general formulae (Ia) and (Ib).

4. A process as claimed in any of claims 1 to 3, characterized in that
5 to 40% by weight and, more particularly, 10 to 20% by weight of amines and
95 to 60% by weight and, more particularly, 90 to 80% by weight of water and/or auxiliaries and additives,
based in either case on the formulation as a whole, are used.

5. A process as claimed in any of claim 4, characterized in that solubilizers are used as the auxiliaries or additives.

6. A process as claimed in claim 4, characterized in that nonionic and/or anionic surfactants are used as auxiliaries or additives.

7. A process as claimed in any of claims 1 to 6, characterized in that a pH value in the range from 4 to 11 and preferably in the range from 5 to 8 is adjusted.

8. A process as claimed in any of claims 1 to 6, characterized in that a dynamic viscosity of less than 300 mPa.s and preferably in the range from 20 to 100 mPa.s is adjusted.

9. A process as claimed in any of claims 1 to 8, characterized in that
a) 2 to 10% by weight of a secondary amine corresponding to general formulae (IIb) and/or (IIc),
b) 2 to 10% by weight of a tertiary amine corresponding to general formulae (IIIb) and/or (IVb),
c) for the rest, water and optionally auxiliaries and additives,
are used, components a and b being present in a ratio by weight of a to b of 1:2 to 3:1 and preferably 2:1.

10. A process as claimed in any of claims 1 to 9, characterized in that beverage bottles, more particularly glass bottles or plastic bottles, cans, glasses, barrels and/or beverage containers, are sprayed as the empties.

## Revendications

1. Procédé pour la désinfection et le nettoyage dans l'industrie alimentaire et dans l'industrie des boissons, en utilisant des amines, caractérisé en ce qu'on utilise un lubrifiant connu en soi à base d'amines secondaires et/ou tertiaires et/ou de sels d'amines de ce type et éventuellement des diluants ou encore des adjuvants, respectivement des additifs habituels pour soumettre à une aspersion superficielle des conditionnements vides, qui contient
(a) au moins une amine secondaire répondant aux formules générales (Ia) ou (Ib)
R-NH-R¹ (Ia)
R-N⁺H₂-R¹X⁻ (Ib)
et/ou
(b) au moins une diamine secondaire répondant aux formules générales (IIa), (IIb) ou (IIc)
R-NH-(CH₂)₃NH₂ (IIa)
R-NH-(CH₂)₃N⁺H₃ X⁻ (IIb)
R-N⁺H₂-(CH₂)₃-N⁺H₃ 2X⁻ (IIc)
et/ou
(c) au moins une amine tertiaire répondant aux formules générales (IIIa) ou (IIIb)
R-NR³R⁴ (IIIa)
R-N⁺HR³R⁴ X⁻ (IIIb)
les radicaux R et R¹ représentant respectivement, indépendamment l'un de l'autre :
- un radical alkyle substitué ou non substitué, linéaire ou ramifié, saturé ou bien une fois ou plusieurs fois insaturé, contenant de 6 à 22 atomes de carbone, qui peut présenter, comme substituant, au moins un radical amine, un radical imine, un radical hydroxyle, un radical halogène et/ou un radical carboxyle,
- un radical phényle substitué ou non substitué qui peut présenter, comme substituant, au moins un radical amine, un radical imine, un radical hydroxyle, un radical halogène, un radical carboxyle et/ou un radical alkyle linéaire ou ramifié, saturé ou bien une fois ou plusieurs fois insaturé, contenant de 6 à 22 atomes de carbone, et
X⁻ représente un anion choisi parmi le groupe comprenant les amidosulfonates, les nitrates, les halogénures, les sulfates, les hydrogénocarbonates, les carbonates, les phosphates ou
R²-COO⁻,
formule dans laquelle le radical R² représente
- un atome d'hydrogène,
- un radical alkyle contenant de 1 à 20 atomes de carbone ou un radical alcényle contenant de 2 à 20 atomes de carbone, substitués ou non substitués, linéaires ou ramifiés, qui peuvent présenter, comme substituants, au moins un radical hydroxyle, un radical amine ou un radical imine, ou
- un radical phényle substitué ou non substitué qui peut présenter, comme substituant, un radical alkyle contenant de 1 à 20 atomes de carbone,
et dans lesquelles les radicaux R³ et R⁴ représentent respectivement, indépendamment l'un de l'autre :
- un radical alkyle contenant de 1 à 20 atomes de carbone ou un radical alcényle contenant de 2 à 20 atomes de carbone, substitués ou non substitués, linéaires ou ramifiés, qui peuvent présenter, comme substituants, au moins un radical hydroxyle, un radical amine ou un radical imine, ou
- un radical phényle substitué ou non substitué qui peut présenter, comme substituant, un radical alkyle contenant de 1 à 20 atomes de carbone,
et la fraction des amines s'élevant de 1 à 100% en poids de la formulation totale.

2. Procédé selon la revendication 1, caractérisé en ce que les radicaux R, R¹, R², R³, R⁴ et X⁻, dans les formules générales, présentent les significations ci-après :
- R et R¹ représentent respectivement, indépendamment l'un de l'autre, un radical alkyle linéaire ou ramifié, saturé ou bien une fois ou plusieurs fois insaturé, contenant de 12 à 18 atomes de carbone,
- R³ et R⁴ représentent respectivement, indépendamment l'un de l'autre, un radical alkyle contenant de 1 à 6 atomes de carbone ou un radical alcényle contenant de 2 à 6 atomes de carbone, linéaires ou ramifiés,
- X⁻ représente le radical R²-COO⁻ où R² représente un atome d'hydrogène, CH₃-, HO-CH₂- ou encore CH₃-CH(OH)-.

3. Procédé selon l'une quelconque des revendications 1 et 2, caractérisé en ce qu'on met en oeuvre au moins une amine tertiaire répondant à la formule générale (IVa) ou (IVb)
R-N(CH₃)₂ (IVa)
R-N⁺H(CH₃)₂ X⁻ (IVb)
dans lesquelles les radicaux R et X⁻ ont respectivement les significations indiquées pour les formules générales (Ia) et (Ib).

4. Procédé selon l'une quelconque des revendications 1 à 3, caractérisé en ce qu'on met en oeuvre
de 5 à 40% en poids, en particulier de 10 à 20% en poids d'amines et
de 95 à 60% en poids, en particulier de 90 à 80% en poids d'eau et/ou d'adjuvants ou encore d'additifs,
chaque fois rapportés à la formulation totale.

5. Procédé selon la revendication 4, caractérisé en ce que, comme adjuvant ou comme additif, on met en oeuvre un agent de solubilisation.

6. Procédé selon la revendication 4, caractérisé en ce que, comme adjuvant ou comme additif, on met en oeuvre des agents tensioactifs non ioniques et/ou anioniques.

7. Procédé selon l'une quelconque des revendications 1 à 6, caractérisé en ce qu'on règle une valeur de pH dans le domaine de 4 à 11, de préférence dans le domaine de 5 à 8.

8. Procédé selon l'une quelconque des revendications 1 à 6, caractérisé en ce qu'on règle une viscosité dynamique inférieure à 300 mPa.s, de préférence dans le domaine de 20 à 100 mPa.s.

9. Procédé selon l'une quelconque des revendications 1 à 8, caractérisé en ce qu'on met en oeuvre
a) de 2 à 10% en poids d'une amine secondaire répondant aux formules générales (IIb) et/ou (IIc),
b) de 2 à 10% en poids d'une amine tertiaire répondant aux formules générales (IIIb) et/ou (IVb),
c) le reste étant de l'eau et éventuellement des adjuvants, respectivement des additifs,
les composants a et b étant présents dans un rapport pondéral a:b de 1:2 à 3:1, de préférence de 2:1.

10. Procédé selon l'une quelconque des revendications 1 à 9, caractérisé en ce qu'on soumet à une aspersion superficielle, comme conditionnements vides, des bouteilles destinées à des boissons, en particulier des bouteilles en verre ou des bouteilles en matière synthétique, des boîtes, des verres, des tonneaux et/ou des conteneurs destinés à des boissons.
